Europäisches Patentamt

European Patent Office

Office européen des brevets

(19)

(11) Publication number: **0 170 441**
**B1**

# EUROPEAN PATENT SPECIFICATION

(12)

(45) Date of publication of patent specification: **07.09.88**

(21) Application number: **85304872.6**

(22) Date of filing: **08.07.85**

(51) Int. Cl.⁴: **H 01 B 7/34,** H 01 B 7/28,
H 01 B 3/10

(54) Electrical wire and cable.

(30) Priority: **08.07.84 IL 72333**
**09.07.84 GB 8417504**
**14.01.85 GB 8500817**

(43) Date of publication of application:
**05.02.86 Bulletin 86/06**

(45) Publication of the grant of the patent:
**07.09.88 Bulletin 88/36**

(84) Designated Contracting States:
**AT BE CH DE FR GB IT LI NL SE**

(56) References cited:
EP-A-0 132 343
CH-A-587 548
GB-A-1 133 333

(73) Proprietor: **RAYCHEM LIMITED**
**Rolls House 7, Rolls Buildings Fetter Lane**
**London, EC4 1NL (GB)**

(72) Inventor: **O'Brien, James Martin**
**1 Osprey Close Swindon**
**Wiltshire (GB)**
Inventor: **Penneck, Richard John**
**"Treeve" Westway**
**Lechlade Gloucestershire (GB)**
Inventor: **Duckworth, Stephen John**
**5 Austen Crescent Liden**
**Swindon Wiltshire (GB)**
Inventor: **Smith, Nicholas John Gregg**
**50 Austen crescent Liden**
**Swindon Wiltshire (GB)**

(74) Representative: **Dlugosz, Anthony Charles et al**
**Raychem Limited Intellectual Property Law**
**Department Faraday Road Dorcan**
**Swindon, Wiltshire (GB)**

Courier Press, Leamington Spa, England.

# 0 170 441

## Description

This invention relates to electrical wire and cables and to electrical conductors suitable for use therein.

Numerous forms of electrical cable have been proposed for use in environments where there is a risk of fire and accordingly where fire retardency of the cable is required. These cables may make use of specific, highly effective, halogenated polymers or flame retardant materials such as polytetrafluoroethylene, polyvinyl chloride, or polyvinylidine fluoride as polymers or decabromodiphenyl ether as flame retardant additives. Halogenated systems, however, suffer from the disadvantage that when they are heated to high temperatures during a fire, the liberate toxic and corrosive gases such as hydrogen halides, and a number of halogen free insulating compositions have therefore been proposed, for example in U.S. patent specification No. 4,322,575 to Skipper and in U.K. patent specification Nos. 1,603,205 and 2,068,347A, the disclosures of which are incorporated herein by reference.

In certain fields where cables are used, for example in military, marine or mass transit applications, it is desired to use cables which are capable of functioning at relatively high temperatures. In other instances it is desired to use cables which not only do not burn, or, if they burn, do not liberate toxic or corrosive gases, but also are capable of functioning after having been subjected to a fire, or preferably for a period of time during a fire without shorting or otherwise failing. Cables that are capable of functioning for a period of time during a fire have been called circuit integrity cables or signal integrity cables depending on their use. The previously proposed circuit and signal integrity cables have generally used the principle that the individual conductors should be separated from one another by mica tapes or by large volumes of packing materials or silicones or by combinations thereof in order to prevent the formation of short circuits during a fire, with the result that the previously proposed cables are relatively heavy or large or both. There is therefore a need for a cable that will function at relatively high temperatures or will function after it has been subjected to a fire, and which preferably will retain its integrity for a period of time during a fire but which is smaller or lighter than the previously proposed cables.

According to one aspect, the present invention provides an electrical wire having a conductor which comprises a bundle of copper strands, the bundle having an adherent, electrically insulating, refractory coating which extends around the circumference of the bundle but not around the individual strands, and the individual strands having an intermediate layer extending around them, the intermediate layer being formed from a metal which acts as a barrier to diffusion of oxygen or copper or both and having a thickness of at least 1.5 micrometres.

The intermediate metallic layer may be formed on the copper strands in a number of ways, for instance by electroplating, standard wire cladding techniques such as roll bonding, and by vacuum deposition techniques e.g. sputtering, evaporation, flame spraying, plasma assisted chemical vapour deposition (CVD) or other techniques.

The wire according to the invention is particularly suitable for forming signal integrity cables and circuit integrity cables because, depending on the construction of the wire, when a portion of the cable is subjected to a fire, the refractory coating will provide sufficient insulation between the conductors to enable the cable to operate for a significant length of time even when any polymeric insulation of the wire has been lost.

An additional advantage of the wire and cable according to the invention is that it is very flexible as compared with other signal and circuit integrity cables. The ability of the wire to be bent around very tight bends (small bend radii) without deleterious effect is partly due to the fact that the layer providing the integrity is thinner than with other signal and circuit integrity cables. However, when the conductor is a stranded conductor it may be bent around extremely tight bends without undue stress on the surface of the strands because the strands are displaced from a regular hexagonal packing at the apex of the bend thereby exposing uncoated areas of the strands to the eye. It is highly surprising that even though uncoated strands may be exposed when the wire conductor is bent there is no electrical contact between adjacent stranded conductors after the polymeric insulation has been removed. It is believed that in this case the integrity is retained because the profile of a stranded conductor is not cylindrical but rather is in the form of a hexagon that rotates along the length of the conductors, so that adjacent stranded conductors will touch one another only at a few points along their length, which points are always provided by the outwardly oriented part of the surface of the strands in the outer layer of the conductors. It is these points of contact that are always provided with the refractory coating.

The fact that the refractory coating does not extend around the individual conductors has the additional advantage that the inner strand electrical contact is retained and the dimensions of the bundle are kept to a minimum (since the thickness of the coating may constitute a significant proportion of the strand dimensions for fine gauge conductors) and also it aids the formation of good electrical connections, e.g. crimp connections, to the conductor because a large proportion of the surface of the strands, and the entire surface of the strands in the central region of the conductor, will be uncoated.

The provision of the metallic intermediate layer in the wire according to the present invention overcomes a problem that has been associated with attempts to form high temperature wires using refractory coatings: It has been found that, although the electrical properties of such refractory coatings may be satisfactory, when the articles are exposed to high temperatures, the stability and integrity of the

2

coating can be adversely affected such that the ability of the article to operate for extended periods is severely reduced.

It has been found that wire and cables according to the invention are highly resistant to high temperatures and that the integrity of the refractory coating is not destroyed at high temperatures for relatively long periods of time. By examination of articles in accordance with the present invention and articles in which no intermediate metal layer is present, by means of a scanning electron microscope, it has been observed that one failure mechanism of articles having no intermediate layer is through spalling. When articles are provided with a thin intermediate layer, spalling is reduced and failure occurs through a mechanism in which the underlying copper appears to migrate through the refractory layer and appear at the outer surface of the refractory layer, in the form of small globules or a network of "dykes" or in other cases, in the form of "blisters". This form of failure may occur at temperatures as low as 500°C, well below the melting point of copper. The particular reason why this failure occurs is unclear and it is likely that more than one mechanism is responsible for the failure in different cases. One theory as to the failure mechanism is that, at elevated temperatures, the underlying copper is oxidized by ambient oxygen which has penetrated the refractory layer, either by diffusion or through cracks that may have been caused by mechanical or thermal stresses in the fractory layer, to form cuprous oxide ($Cu_2O$ or $CuO$ which are relatively electrically conductive. Growth of the copper oxide scale would by outward diffusion of copper through copper oxide to combine with inwardly diffusing oxygen until it reached the outer surface of the refractory layer. In the case of circuit integrity wires electrical integrity of the system would be significantly deleteriously affected.

In the case of a stranded conductor in which the conductor bundle (rather than the individual strands) is provided with a refractory coating, the problem of growth of cuprous oxide scale is particularly severe since it is inevitable that some cracking of the refractory coating will occur in the regions between the strands and ambient air will be able to penetrate into the interstices of the strands which, in turn, causes cuprous oxide scale to swell out of the conductor between the strands. This problem is overcome or substantially reduced according to the invention by providing an intermediate metal layer around the individual strands, which acts as a barrier to diffusion of oxygen or copper or both.

It has been found that, in articles according to the invention the metal forming the intermediate layer eliminates or substantially reduces the mechanisms by which failure occurs, thus extending the high temperature lifetime of the article. Thus, for example in the case of circuit or signal integrity cables the time required to cause circuit failure in a fire would be substantially increased. The metal forming the intermediate layer for this purpose may be one which acts as a barrier to diffusion of either the underlying substrate to the outer surface of the article or to the diffusion of oxygen into the substrate. It may, restrict diffusion in its elemental form or it may hinder diffusion processes, by formation of oxide scales when exposed to air, as is the case with for example aluminium or nickel. Such scales are most effective if they are stable on formation and exhibit low growth rates. The intermediate layer may be formed of metals which will alloy with the underlying substrate on exposure to high temperatures but which would still preferentially oxidise to form stable scales on exposure to air, or may be formed from metallic alloys which exhibit high oxidative stability e.g. titanium/aluminium or nickel/chromium alloys. The metal forming the intermediate layer may also be selected to take advantage of physical or chemical compatibility with the substrate and refractory layers to maximise adhesion.

The preferred metals for use in the intermediate layer include aluminium, titanium, nickel, chromium, manganese, tin and silver, especially nickel.

In certain cases the ability of the wire to withstand high temperatures may be substantially improved by the provision of a further metallic layer between the intermediate layer and the refractory layer. For example the temperature resistance of a wire having a nickel intermediate layer and an alumina refractory coating may be considerably increased by interposing an additional layer of aluminium between the nickel and the alumina. It has been observed that the improvement is due to a further reduction of the copper migration described above. Thus, according to another aspect, the present invention provides an electrical wire having a conductor which comprises a bundle of copper strands, the bundle having an adherent, electrically insulating, refractory coating that extends around the bundle but not around the individual strands, and the individual strands having an intermediate layer extending around them, the intermediate layer being formed from a metal which acts as a barrier to diffusion of oxygen or copper or both, the conductor including an additional metallic layer between the intermediate layer and the refractory coating. It has also been observed that the provision of a relatively thick intermediate layer and/or further metallic layer can act to reduce or eliminate crack formation resulting from the thermal expansion mismatch between the copper and the refractory layer.

The additional metallic layer may, if desired, extend around the individual strands of the conductors or it may be, and preferably is, present only in those areas that are provided with the refractory coating. For example, it is preferably provided around the conductor only when the individual strands have been laid up to form the stranded conductor. This layer may be applied in the same way as the other metallic intermediate layer although the particular method that is used may depend on whether the additional layer extends around the individual strands or around the bundle as a whole. Where the additional layer extends round the bundle as a whole vacuum deposition techniques e.g. sputter plating are preferred. In this case the intermediate layer and the additional layer may comprise the same metal, so that each strand is enclosed within a metal layer that is thicker on the outwardly facing surfaces of the outer strands. The preferred metal for the intermediate layer in this construction is nickel and for the additional layer, aluminium or nickel are preferred.

In addition it has been found that in many cases the provision of a relatively thick intermediate and/or further metallic layer (for example formed from aluminium) can significantly reduce the formation of cracks in the refractory layer when the article is subjected to mechanical abuse. It is believed that the reduction in formation of cracks is due to the reduction of stress in the refractory layer when the article is subjected to strain by virtue of the deformation of the intermediate layer. Thus, the wire according to the invention advantageously includes an intermediate and/or further metallic layer formed from a metal having a lower modulus than that of copper. Since the absolute value of the modulus will depend on the strain, for strains beyond the limit of proportionality, and on the morphology of the material, the modulus as used herein refers to an arbitary value of 1% strain and for the annealed material in its bulk form.

The intermediate layer and/or any additional metallic layer preferably each has a thickness of at least 0.3, more preferably at least 1, especially at least 1.5 and most especially at least 3 micrometres, the temperature stability of the refractory layer increasing markedly with increasing thickness of the intermediate and/or additional layer.

The refractory coating may, in the broadest aspect of the invention, be applied to the intermediate layer by any of a number of techniques. For example a metal layer may be deposited and then oxidized, e.g. an aluminium layer may be formed on the strands and then, when the strands have been laid up to form the stranded conductor, it may be oxidized. However it is preferred for the refractory layer to be substantially contaminant-free, that is to say, for the refractory layer to contain only those species that are intended in order for the layer to fulfill its intended function, and contains substantially no species that result from the manufacturing process. An important feature of the refractory layer is good control of composition to optimise the high temperature performance of the article. The refractory composition is totally inorganic and therefore does not rely on conversion processes to occur during exposure to normal or emergency high temperature service, as is the case for example in many mica filled or glass filled silicone resin systems. The composition is also improved by removing the use of polymeric binders to support inorganic materials which may be consolidated by firing processes to form the inorganic insulation. Similarly, articles in which the refractory coatings have been formed by electrochemical conversion of metal layers e.g. by anodising an aluminium layer, are not preferred, such layers often being heavily contaminated with ionic residue from the electrolytic solutions e.g. sulphates from sulphuric acid anodisation processes. Such wet chemical processes may also attack areas of the article not protected by the metal layer undergoing the process and may result in contamination of the intermediate layer described above. Preferred deposition techniques include physical vapour deposition processes such as reactive evaporation and sputtering or plasma assisted chemical vapour deposition. Coatings can also be formed by plasma oxidation of the metals or by non vacuum processed such as a high pressure CVD method.

The refractory coating preferably has a thickness of at least 0.5, more preferably at least 1 and especially at least 2 micrometres but preferably not more than 15 and especially not more than 10 micrometres, the most preferred thickness being about 5 micrometres depending upon specific operational requirements. The exact thickness desired will depend on a number of factors including the type of layer and the voltage rating of the wire, circuit integrity cables usually requiring a somewhat thicker coating than signal integrity cables and sometimes above 15 micrometres. The lower limits for the coating thickness are usually determined by the required voltage rating of the wire whilst the upper limits are usually determined by the time, and therefore the cost, of the coating operation.

Preferably the insulating refractory coating is formed from an electrically insulating infusible or refractory metal or semi-metal oxide or nitride and the invention will be described below in many cases with respect to oxides and nitrides although other refractory coatings are included. By the term "infusible" or "refractory" is meant that the coating material in its bulk form should not fuse or decompose when subjected to a temperature of 800°C, for 3 hours. Preferably the oxide or nitride should be able to withstand higher temperatures also, for example it should be able to withstand a temperature of 1000°C for at least 20 to 30 minutes. The preferred oxides are those of aluminium, titanium, tantalum and silicon or mixtures thereof with themselves or with other oxides and the preferred nitrides are those of aluminium and silicon. Thus, for example, the use of mixed metal oxides for the refractory coating are also encompassed by the present invention. It should be appreciated that the oxide or nitride layer need not, and in many cases will not, have a precisely defined stoichiometry. In a number of cases, depending on the method of forming the refractory coating, the coating will contain the metal or semi-metal in a stoichiometric excess, that is to say, the coating will contain more metal than is required for the stoichiometry of a defined formal oxidation state of the metal. Accordingly the terms "aluminium oxide", "titanium oxide", "tantalum oxide", "silicon oxide", "metal oxide" and the equivalent terms when referring to nitrides are intended to include non-stoichiometric compounds. It is often advantageous for the refractory coating to be non-stoichiometric since this may increase the adhesion between the refractory coating and the underlying layer, and especially if the stoichiometry of the refractory coating varies through at least part of its thickness so that stresses that may be induced in the coating, for example due to differential thermal expansion, are not localised to a boundary of the coating and so that different parts of the coating will exhibit different properties. For example, a relatively metal-rich part of the coating may exhibit good adhesion to the

4

conductor or intermediate layer while part of the coating having least metal or semi-metal may exhibit the best electrical properties.

If desired, the stoichiometry of the refractory coating may vary continuously throughout the thickness of the coating or it may contain one or more layers or coating may have an outer region of relatively uniform stoichiometry and preferably of a relatively high oxygen or nitrogen content in order to exhibit the optimum electrical properties. The relative thicknesses of the non-uniform and uniform layers may vary widely. For example the major part of the coating may have a non-uniform stoichiometry or the major part of the coating's thickness may be of uniform stoichiometry, in which latter case the non-uniform part of the coating could even be considered as an intermediate layer that improves adhesion of the coating especially at high temperatures. If the underlying metal or semi-metal-rich part of the coating is intended to improve the adhesion of the refractory coating, its particular composition will depend on the composition of any underlying layer, and in some cases it may be desirable for the metal or semi-metal rich part to consist substantially entirely of the metal or semi-metal so that there is a gradual change from the metal or semi-metal to the oxide or nitride. This is particularly preferred if the system includes an intermediate layer of the same metal- or semi-metal.

The precise stoichiometry of the uniform top layer can be determined experimentally using wavelength dispersive electron microprobe analysis or by using x-ray photoelectron spectroscopy (XPS). The composition of the coating as it changes from metal to refractory throughout its depth can be assessed using Auger electron spectroscopy (AES) in which the film is continuously sputtered away to expose fresh surfaces for composition analysis.

The variation in stoichiometry is not limited to a variation in the metal or semi-metal/oxygen or nitrogen proportions. In addition or alternatively the relative proportions of two different metals or semi-metals may be varied so that, for example, there is a gradual change from one metal, which may constitute an intermediate layer, to the oxide or nitride of a different metal.

The outer region of the refractory coating preferably has a molar oxygen or nitrogen content that is at least 50%, more preferably at least 65% and especially at least 80% of the oxygen or nitrogen content of that required for the formal stoichiometry of the insulating refractory oxide or nitride. Thus the preferred oxide composition of the outer region may be represented as $MO_x$ where x is

at least 0.75, preferably at least 1 and especially at least 1.25 in the case of aluminium,

at least 1, preferably at least 1.3 and especially at least 1.5 in the case of titanium or silicon, and

at least 1.25, preferably at least 1.6 and especially at least 2 in the case of tantalum.

For relatively thin refractory coatings that have a stoichiometric excess of the metal or semi-metal it has been found that the coating remains insulating as the temperature is raised up to a certain temperature, usually in the range of 300 to 600°C and then becomes conductive when a load of 30 V is applied. In general the electrical properties of the coatings, as determined by the temperature of onset of conductivity, may be improved both by increasing the thickness of the coating and by increasing the oxygen or nitrogen content thereof although to some extent either the thickness or the oxygen or nitrogen content may be increased at the expense of the other.

Although it is possible, at least in the broadest aspect of the invention, for the refractory coating to consist of a single layer only which is deposited on the intermediate layer it is possible, and in many cases preferable, for one or more additional layers to be formed. For example a refractory coating comprising an oxide may have a refractory nitride layer thereon. Examples of nitrides that may be deposited on refractory coatings to improve the mechanical properties include titanium nitride or aluminium nitride.

In the case of wires according to the invention, polymeric insulation may be provided in order to provide additional insulation to the conductor during normal service conditions and also to enable the wire to have the desired dielectric properties and other properties e.g. mechanical properties, scuff resistance, colour coding ability etc. However, an important advantage of the present invention is that since a significant proportion of or all the service insulating properties are provided by the refractory coating, the electrical properties of the polymeric insulation are not as critical as with other wire constructions in which the polymeric insulation provides the sole insulation between the conductors. Of the known polymeric materials that are used for electrical insulation, polyethylene probably has the most suitable electrical properties but is highly flammable, and has poor mechanical properties. Attempts to flame retard polyethylene have either required halogenated flame retardants which, by their nature, liberate corrosive and toxic hydrogen halides when subjected to fire, or have required relatively large quantities of halogen-free flame retardants which have a deleterious effect on the electrical properties and often also the mechanical properties of the polymer. Accordingly, an acceptable wire has in the past only been achieved by a compromise between different properties which is often resolved by using a relatively thick-walled polymeric insulation and/or dual wall constructions. Although such forms of polymeric insulation may be used with the wire according to the present invention, the presence of the refractory layer does obviate these problems to a large extent since the polymer used for the insulation may be chosen or its flammability and/or its mechanical properties at the expense of its electrical properties. As examples of polymers that may be used to form the polymeric insulation there may be mentioned polyolefins e.g. ethylene homopolymers and copolymers with alpha olefins, halogenated polymers e.g. tetrafluoroethylene, vinylidene fluoride, hexafluoropropylene and vinyl chloride homo or copolymers of polyamides, polyesters, polyimides, polyether ketones e.g. polyarylether ketones, aromatic polyether imides and sulphones, silicones, alkene/vinyl acetate copolymers and the like. The polymers may be used alone or as blends with one another and may contain fillers e.g. silica and metal oxides e.g. treated and untreated metal oxide flame retardants such as hydrated alumina and titania. The polymers may be used in single wall constructions or in multiple wall constructions, for example a polyvinylidine fluoride layer may be located on for example a polyethylene layer. The polymers may be uncrosslinked but preferably are crosslinked, for example by chemical cross-linking agents or by electron or gamma irradiation, in order to improve their mechanical properties and to reduce flowing when heated. They may also contain other materials e.g. antioxidants, stabilizers, crosslinking promotors, processing aids and the like. It is particularly preferred for the polymeric insulation to contain a filler e.g. hydrated alumina, hydrated titania, dawsonite, silica and the like, and especially a filler that has the same chemical composition, at least under pyrolysis conditions, as the refractory coating, so that the filler in the polymeric insulation will provide additional insulation when the wire or cable is subjected to a fire. Another preferred type of polymeric insulation is one that will char, for instance certain aromatic polymers mentioned above, or that will ash e.g. a silicone polymer, when subjected to a fire so that the char or ash, together with the refractory coating, will provide the necessary insulation during a fire. Examples of polymers, compositions, their manufacture and wires using them are described in U.S. Patent Specifications Nos. 3,269,862, 3,580,829, 3,953,400, 3,956,240, 4,155,823, 4,121,001 and 4,320,224, British Patent Specifications Nos. 1,473,972, 1,603,205, 2,068,347 and 2,035,333, 1,604,405 and in European Patent Specification No. 69,598, the disclosures of which are incorporated herein by reference. In some instances, for example when certain aromatic polymers are used it may be appropriate to form the insulation on the conductor by a plasma or thermal polymerisation process. Preferably the wire is substantially halogen free.

A vacuum deposition method such as evaporation, plasma assisted chemical vapour deposition, or especially a sputtering method is preferred.

In the sputtering method, predominantly neutral atomic or molecular species are ejected from a target, which may be formed from the material to be deposited, under the bombardment of inert gas positive ions e.g. argon ions. The high energy species ejected will travel considerable distances to be deposited on the wire conductor substrate held in a medium vacuum, e.g. $10^{-4}$ to $10^{-2}$ mbar. The positive ions required for bombardment may be generated in a glow discharge where the sputtering target serves as the cathode electrode to the glow discharge system. The negative potential (with respect to ground and the glow discharge) is maintained in the case of insulating target materials by the use of radio frequency power applied to the cathode, which maintains the target surface at a negative potential throughout the process. DC power may be applied when the target is an electrically conducting material. The advantage of such techniques is that control of the target material is greatly enhanced, and the energy of the species ejected is very much higher than with evaporation methods e.g. typically 1 to 10 eV for sputtering as compared with 0.1 to 0.5 eV for evaporation methods. Considerable improvements in interfacial bonding are achieved but the deposition rate in the sputtering process described will be lower than that for electron beam evaporation.

In magnetron sputtering processes the plasma is concentrated immediately in front of the cathode (target) by means of a magnetic field. The effect of the magnetic field on the gas discharge is dramatic. In that area of discharge where permanent magnets, usually installed behind the cathode, create a sufficiently strong magnetic field vertically to the electric field, secondary electrons resulting from the sputter bombardment process will be deflected by means of the Lorenz force into circular or helical paths. Thus the density of electrons immediately in front of the cathode as well as the number of ionised argon atoms bombarding the cathode are substantially increased. There is an increase in plasma density and a considerable increase in deposition rate. Bias sputtering (or sputter ion plating) may be employed as a variation of this technique. In this case the wire conductor is held at a negative potential relative to the chamber and plasma. The bombardment of the wire conductor by Argon ions results in highly cleaned surfaces. Sputtering of the target material onto the wire conductor throughout this process results in a simultaneous deposition/cleaning mechanism. This has the advantage that the interfacial bonding is considerably improved. In sputter ion plating systems both substrate and the wire conductor are held at a negative potential. In this case the relative potentials are balanced to promote preferential sputtering of the target material. The target voltage will be typically less than 1 KV, dependent on system design and target material. The wire substrate, may be immersed in its own localised plasma dependent upon its bias potential, which will be lower than that of the target. The exact voltage/power relationship achieved at either target or substrate is dependant upon many variables and will differ in detail from system to system. Typical power densities on the target are 10—20 W/cm². The load to the substrate may be substantially lower, often as little as 5% of the target load.

The preferred technique that is used to apply the oxide or nitride coating is a reactive bias sputtering method in which reactive gas is introduced into the vacuum chamber in addition to argon so that the oxide/nitride of the target material, which in this case, is a metal rather than the oxide/nitride will be deposited. Experimental results have shown that the level of reactive gas and its admission rate have a significant effect on deposition rates. The precision control of partial pressure of the reactive gas and the analysis of the sputtering atmosphere in a closed loop control system is considered highly desirable. Apart from the simultaneous deposition/cleaning advantages mentioned above, the ion bombardment of the

6

substrate enhances surface reaction between the reactive gas and depositing species, resulting in more efficient formation of the coating with the required stoichiometry.

Partial pressure of reactive gas is determined experimentally but will normally be between 2 and 25% but sometimes up to 30%, the exact level depending on the required stoichiometry of the coating and deposition rate. Reactive sputtering is also the preferred technique because it facilitates alterations to the stoichiometry of the coating. For example, an intermediate "layer" of the pure metal used for the oxide/nitride coating may be deposited in such a way that there is no defined boundary between the conductor metal, oxide/nitride metal and oxide/nitride layers.

The vacuum chambers and ancillary equipment, including micro-processor gas control units and a variety of targets used in these methods may be purchased commercially. Many variations in design are possible but most employ the use of "box" shaped chambers which can be pumped down to high vacuum for use in any of the vacuum deposition processes mentioned.

Systems are normally, but not exclusively, dedicated to one deposition process. One system which may be employed to coat wire uses air to air transfer techniques for passage of the wire conductor through the deposition chambers and employs one or more ancillary vacuum chambers either side of the main deposition chamber.

These ancillary chambers are held at progressively higher pressures as they extend from deposition chamber to air. This reduces the load on individual vacuum seals. The system described has the advantage of continuous feed of the wire conductor over batch process arrangements. In the vacuum deposition chamber the pressure is held constant at a pressure normally between $10^{-4}$ and $10^{-2}$ Torr ($1,33 \cdot 10^{-4}$ and $1,33 \cdot 10^{-2}$ mbar).

The targets employed are commercially available Planar Magetron Sputtering sources. Their size may vary widely, and targets in excess of 2 metres in length may be employed. Between two and four such sources may be arranged opposite one another so as to surround the wire conductor passing through the chamber or to sputter from at least two sides. The arrangement may be employed in series to increase wire throughput rates. As described above a negative bias is applied to the magnetron to initiate the sputtering process. The wire may be held at a lower negative bias as described earlier.

Refinements to the system can, if desired, be employed. For example, the use of an intermediate vacuum station between the air (input side) and the deposition chamber may be employed to generate an Argon ion glow discharge which cleans the wire conductor surface by ion bombardment prior to its entry into the vacuum deposition chamber and also heats the wire conductor.

Further intermediate chambers can be employed between the cleaning and deposition chamber to deposit intermediate layers.

Conditions may be controlled to produce any of the conductor coatings described above in which no defined boundaries occur between the layers. For example an intermediate "layer" of the pure metal used for the refractory coating may be deposited in such a way that there is no defined boundary between the conductor metal, the intermediate layer and the oxide or nitride coating. In a similar fashion additional chambers can be employed between the deposition chamber and air (output side) to deposit different metal, metal oxide or metal alloys onto the refractory coating for improved lubrication or wear resistance.

Evaporation and the related processes of activated evaporation and ion plating offer alternative techniques for deposition of the coating, with significant advantages in deposition rate.

Evaporation of the coating material is achieved by heating the material such that its vapour pressure exceeds $10^{-2}$ mbar. Evaporation temperatures vary according to coating material, e.g. 1300—1800°C for refractory metal oxides, the pressure being usually $10^{-4}$ to $10^{-6}$ mbar. Similar wire transport system to those described may be used to hold the substrate about 30—40 cm above the source. Several heating methods exist e.g. resistive, inductive, electron beam impingement etc. although the preferred method is an electron beam source where a beam of high voltage electrons e.g. at a potential of 10,000 V impinge onto the coating material contained in a water-cooled crucible. The use of multi-pot crucibles or twin source guns, enable multiple layers and graded stoichiometry layers to be deposited with the aid of electronic monitoring and control equipment.

Compound coatings can be made either by direct evaporation from that compound e.g. $Al_2O_3$ or by reactive evaporation, e.g. aluminium evaporated into a partial pressure of oxygen to give aluminium oxide. Variations in the process exist either to promote reactions or adhesion, e.g. Activated reactive evaporation (ARE) can be used to increase the reaction probably between the evaporant and the reactive gas.

In ion-plating, negative bias applied to the substrate in an inert gas, promotes simultaneous cleaning/deposition mechanisms for optimising adhesion as described in the sputtering process. Bias level of −2 KV are typically used but these can be reduced to suit wire substrates. Alternatively, high bias can be applied to a plate positioned behind the traverse wire to achieve a similar effect. As operating pressures are higher in the ion plating technique, e.g. $10^{-3}$ to $10^{-2}$ mbar, gas scattering results in a more even coating distribution. To protect the filament the electron beam gun in the ion plating technique is differentially pumped to maintain vacuum higher than $10^{-4}$ mbar.

In the Plasma assisted chemical vapour deposition (PACVD) method the substrate to be coated is immersed in a low pressure (0.1 to 10 Torr) (0,133 to 13,3 mbar) plasma of the appropriate gases/volatile compounds. This pressure is maintained by balancing the total gas flow-rate against the throughput of the pumping system. The plasma is electrically activated and sustained by coupling the energy from a power generator through a matching network into the gas medium. Thin films have been successfully deposited from direct current and higher frequency plasmas well into the microwave range. At high frequencies the energy may be capacitatively or inductively coupled depending on chamber design and electrode configuration. Typically a 13.56 MHz radio-frequency generator would be used having a rating which would allow a power density of between 0.1—10 W/cm² in a capacitatively-coupled parallel-plate type reactor. The substrate, which could be set at a temperature of up to 400°C, may be grounded, floating at plasma potential or subjected to a dc voltage bias as required. Typically deposition rates for this technique can be favourably compared with those obtained by sputtering. The deposition of alumina may be achieved by immersing a substrate in a plasma containing a volatile aluminium compound (e.g. Tri-methyl aluminium or Aluminium butoxide) and oxygen under appropriate processing conditions.

After the oxide coating has been deposited on the wire conductor the polymeric insulation may be extruded onto the coated conductor by methods well known in the art.

In order to form a circuit or signal integrity on cable the appropriate wires according to the invention may simply be laid together and be enclosed in a jacket. If desired the wires may be provided with a screen or electromagnetic interference shield before the cable jacket is applied. Thus a cable may be formed in a continuous process by means well known in the art by braiding the wire bundle and extruding a cable jacket thereon. Any of the materials described above for the wire polymeric insulation may be used although halogen-free compositions e.g. compositions as described in the U.K. Patent Specifications Nos. 1,603,205 and 2,068,347A mentioned above are preferred. It is of course possible to employ additional means for providing integrity of the cable such as mica tape wraps, but these are not necessary nor are they desirable in view of the increased size and weight of the cable.

In certain circumstances it may be desirable to coat the oxide layer with a thin coating of a polymeric resin or lacquer in order to provide a barrier against water or electrolytes during service.

A further aspect of the invention is an electrical cable which comprises a bundle of wires as described hereinbefore.

The present invention is especially suitable for forming flat cables which, as will be appreciated, are not susceptible to being wrapped with mica tape. A flat cable which comprises a plurality of elongate metallic electrical conductors can be formed which has an adherent coating of an electrically insulating refractory oxide or nitride of a metal or semi-metal other than that from which the conductors, the conductors being laid in side-by-side relationship and enclosed in a continuous polymeric cable insulating layer.

Several embodiments of the invention and a method of production thereof will now be described by way of example with reference to the accompanying drawings in which:

Figure 1 is a cross-section through one form of wire according to the present invention;

Figure 2 is a section through part of the wire of figure 1 showing the wire in greater detail.

Figure 3 is a cross-section through a signal integrity cable employing the wires of figure 1; and

Figure 4 is a schematic view of part of the sputtering apparatus showing its wire handling mechanism.

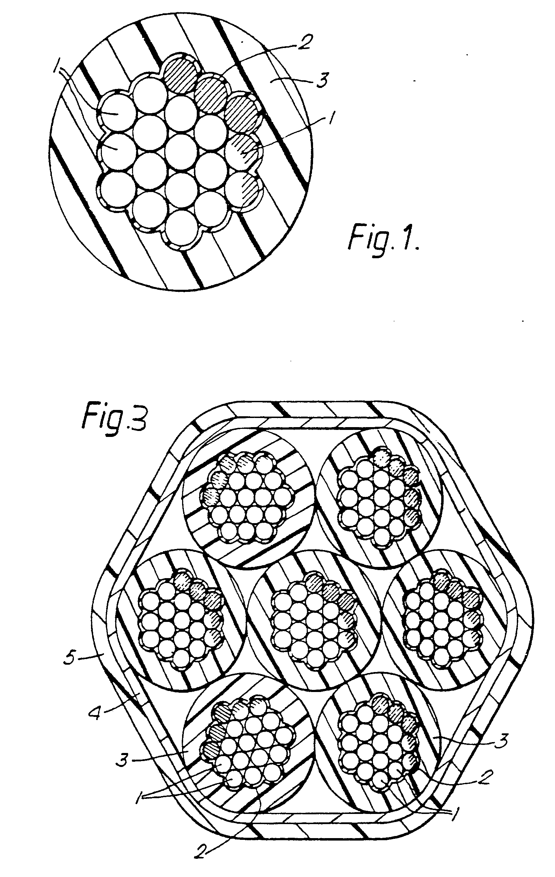

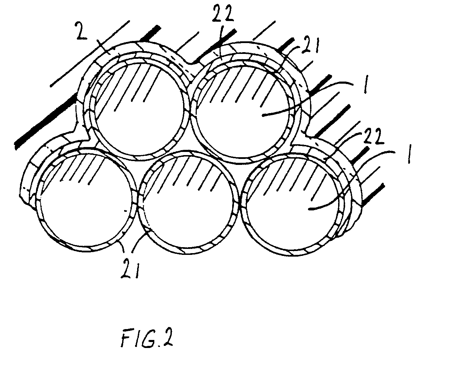

Referring to figures 1 and 2 of the drawings a 26 AWG stranded copper conductor formed from 19 nickel plated copper strands 1 (each having a nickel coating 21 of thickness of approximately 1.5 micrometres) is coated with a 5 micrometre thick layer 2 of aluminium oxide by the reactive sputter ion plating method described above. Before the deposition of the alumina the outer surface of the stranded conductor was provided with a 3 micrometre thick layer of aluminium 22. A coating 3 based on a polyetherimide sold under the trade name "ULTEM" or a polyether ether ketone or polyether ketone is then extruded on the oxide coated conductor to form a polymeric "insulating" layer of mean wall thickness 0.2 mm.

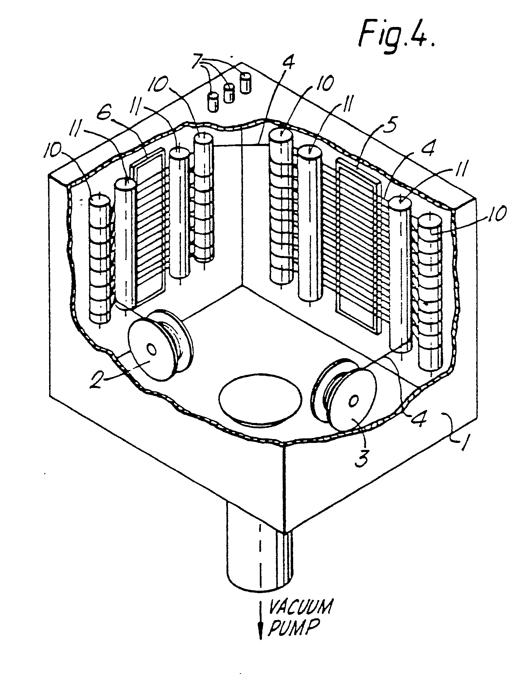

Figure 3 shows a signal integrity cable formed by laying together seven wires shown in figure 1, forming an electromagnetic interference screen 4 about the bundle by braiding and then extruding thereon a jacket 5 based on a halogen-free composition as described in British Patent Specification No. 2,068,347 Example 1A.

The cable so formed is particularly lightweight and has a relatively small overall diameter in relation to the volume of the copper conductor.

Apparatus for use in a batch process for coating wire conductor substrate is illustrated in Fig. 4. The apparatus comprises a vacuum chamber into which a complete wire transport mechanism which includes wire pay-off reel 2 and take-up reel 3, wire support rolls 10 and tensioning rolls 11 is loaded. The mechanism engages motor drives which control the passage of wire 4 so that the wire traverses a vertically mounted target 5 a number of times. Deposition occurs by the processes previously described. As before, variations in set-up are possible. An additional target (not shown) may be employed on the other side of the wire to increase coating rates and additional targets, e.g. target 6 can be employed to deposit intermediate layers before and/or after deposition of the primary oxide/nitride coating. Suitable design of the gas inlet system to suit the specific geometries employed can facilitate deposition of layers which have no defined boundaries as described previously. Batch length will depend on chamber dimensions and transport system design.

In the operation of such a batch process wire 4 is transferred from one reel 2 to the other 3 within the chamber. The route taken by the wire may cause it to pass before the smaller ancillary target 6 to deposit an intermediate layer of any desired material. Power to this target, combined with wire speed and the number of passes in front of the target will control the thickness of the intermediate layer deposit. The wire 4 may then pass in front of the larger primary target 5 to deposit the main coating. Again thickness will be dictated by a combination of power, wire speed and a number of passes. The ratio of thicknesses between the intermediate and the primary coating is controlled in the same way. Multi-layers can be built up by reversing the mechanism as desired such that the wire 4 passes back past the targets 5, 6 in reverse order. Thickness and composition may be altered in the reverse pass as required, e.g. the process employed at the smaller magnetron may be reactive on the reverse pass to deposit a compound of the metal on the intermediate layer, e.g. Ti and $TiN_x$. Deposition of layers with no defined boundary between the metal intermediate layers (or substrates) and the oxide/nitride coatings may be achieved by setting up gradients of reactive gas in front of the primary target, such that wire at the top edge of the target 5 is subjected to deposition in an Argon rich atmosphere which gradually increases in reactive gas content as the wire progresses down the face of the target. A gradient can be achieved by a baffle system (not shown) which progressively leaks oxygen introduced at the bottom end of the target towards the upper end.

A simpler technique for producing the layer with no defined boundary involves use of a multipass process in which wire 4 is passed back and forth through the system, and with each pass the level of reactive gas is increased to a final level required to obtain the correct stoichiometry. Thus the stoichiometry of the intermediate layer increases in a series of small incremental steps from metal to required stoichiometry. Composite targets may also be used to produce intermediate layers with stoichiometry gradients. In the case of discrete articles, the articles may instead be held in front of the target by means of a rotating sample holder.

Examples 1 to 6

19 strand, 22 AWG copper wire which had been conventionally coated 360° around each strand with approximately 1.5 micrometres of nickel, and uncoated copper conductors were provided with aluminium intermediate layers of various thicknesses by use of the sputtering apparatus shown schematically in figure 4 of the drawings. The sputtering conditions were as follows: the wire 4 was precleaned by vapour degreasing in 1,1,1-trichloroethane prior to deposition. The cleaning was achieved by passing the wire through a vapour degreasing bath such that a residence time of 3 minutes was achieved. The wire 4 was then loaded into the vacuum chamber. The chamber was then evacuated to a pressure of $1 \times 10^{-6}$ mbar prior to starting the process. At this stage argon was admitted to attain a pressure of $1.5 \times 10^{-2}$ mbar whereupon a high frequency (80 kHz) bias potential was applied to the wire handling system which was isolated from ground. A bias potential of $-850$ V was achieved, and the wire was transferred from reel 3 to reel 4 such that a residence time of 10 minutes was achieved. On completion of the cleaning cycle the pressure was reduced to $8 \times 10^{-3}$ mbar and the deposition process started.

3 kW of DC power was applied to the aluminium target 5. The wire passed from reel 2 to reel 3 being coated as it passed the target 5. Residence time in this region was controlled by wire speed and adjusted to give the required thicknesses. The roller mechanism alternated the wire face exposed to the target as it progressed down the target length.

Samples of conductor coated with aluminium as described above were susequently coated with aluminium oxide in a similar process. For this second coating, an aluminium oxide target powered with an RF power supply was used. The wire residence time and target power were adjusted to give a constant thickness of aluminium oxide, being about 4 micrometres. During deposition of both aluminium and aluminium oxide the conductors were held at a negative bias potential relative to the chamber to promote adhesion.

The electrical performance of the insulated wires so formed was tested by twisting a pair of identical wires (2 twists per 2.5 cms length) to form a twisted pair cable, connecting one end of the wires to a 1 MHz, 30 V square wave source and observing the wave across a 200 ohm load at the other end of the wires by means of an oscilloscope. The twisted pair cables were subjected to heating in a propane gas burner having a flat flame 8 cm wide. The temperature of the flame just below the twisted pairs was maintained at 900°C and the time to failure recorded.

The results are given in Table 1, from which it may be seen that nickel coated wires, and especially those provided with additional layers beneath the refractory coating according to the invention exhibit significantly greater times to failure:

TABLE 1

| Example | Nickel intermediate layer thickness (micrometres) | Aluminium additional layer thickness (μm) | Time to failure in a 900°C propane flame (mins) |
|---|---|---|---|
| 1 (Comparison) | 0 | 0 | 0.2 |
| 2 | 1.5 | 0 | 19 |
| 3 (Comparison) | 0 | 1 | 2.5 |
| 4 | 1.5 | 1 | 100 |
| 5 (Comparison) | 0 | 3.3 | 32 |
| 6 | 1.5 | 3 | 132* |

Note* Test terminated with no failure noted after 132 minutes

The results of all Examples 1 to 6 clearly illustrate the benefits to performance derived from increasing the thickness of the metallic intermediate layer beneath the refractory insulating layer. A progressive reduction in spalling and cracking of the refractory layer is also noticeable as the thickness of the metal interlayer increases. The additional benefits of coating the individual strands around their entire circumference is clear by comparison of Examples 1, 3 and 5 with Examples 2, 4 and 6 respectively. In Example 4 the total thickness of the metal intermediate layers beneath the refractory layer is less than that used in Example 5 yet a 3 fold improvement in failure time is recorded.

Examples 7 (Comparison) and 8

In Example 7 a 22 AWG stranded copper conductor wire sample was provided with a 3 micrometre thick aluminium layer extending around its circumference (but not around the individual strands) followed by a 4 micrometre thick aluminium oxide layer using the procedure described in Examples 1 to 6.

The sample was then tested by repeatedly passing 36A square wave current pulses of 60 seconds duration through it separated by intervals of 45 seconds. This caused temperature cycling of the samples to a temperature of 750°C. The samples were observed using an optical microscope during the temperature cycling and the formation of copper oxide scale was monitored.

In Example 8, Example 7 was repeated with the exception that the individual strands of the copper conductor had previously been provided with a 1.3 micrometre thick nickel layer. The results are shown in Table 2 from which it can be seen that the provision of the nickel layer around the individual strands considerably improves the resistance of the conductor to oxidation under the temperature cycling.

TABLE 2

| Example | Nickel layer thickness | Al layer thickness | Comments |
|---|---|---|---|
| 7 (Comparison) | — | 3 μm | slight oxide formation after 2 cycles |
| 8 | 1.3 μm | 3 μm | slight oxide formation noted after 100 cycles |

Examples 9 to 13

19 strand 22 AWG copper wires, provided with conventionally-applied 360° nickel intermediate layers around each strand and sputter-coated with additional aluminium layers around the bundle but not around the individual strands, were manufactured to the dimensions shown in table 3. In each case, a sputtered refractory silicon dioxide layer of thickness 5 micrometres was added. Twisted pairs of these wires were tested for their high temperature performance as described above. The results are shown in table 3. Again, the benefits of intermediate and additional layers can be clearly seen.

10

TABLE 3

| Example | Nickel intermediate layer thickness (μm) | Aluminium additional layer thickness (μm) | Time to failure in a 900°C propane flame (min) |
|---|---|---|---|
| 9 (Comparison) | 0 | 0 | 1 |
| 10 | 1.5 | 0 | 13 |
| 11 (Comparison) | 0 | 3 | 3 |
| 12 | 1.5 | 3 | 24 |
| 13 (Comparison) | 0 | 10 | 142 |

Examples 14 to 16

19 strand 22 AWG copper wires, conventionally coated with 360° tin intermediate layers around each strand, and sputter coated with additional aluminium layers around the bundle but not around the individual strands, were manufactured to the dimensions shown in table 4. In each case, a sputtered refractory aluminium oxide layer of thickness 5 micrometres was added. Twisted pairs of these coated wires were tested for their high temperature performance as described in Examples 1—6, except the temperature of the propane flame was adjusted to 750°C. The results are shown in table 4, from which it can be seen that wires provided with intermediate and additional layers according to the invention perform significantly better at high temperatures than those without. The wires of Example 16 were examined with an optical microscope after flame testing. They were seen to be in good condition: there was little cracking of the refractory coating, and copper oxide growth between the strands was minimal.

TABLE 4

| Example | Tin intermediate layer thickness (micrometres) | Aluminium additional layer (micrometres) | Time to failure in a 750°C propane flame (min) |
|---|---|---|---|
| 14 (Comparison) | 0 | 0 | 1 |
| 15 | 1 | 1 | 43 |
| 16 | 1 | 10 | >360* |

* Testing stopped with no failure noted.

Examples 17 and 18

Samples of 19 strand, 22 AWG copper wire, which had been coated with nickel intermediate layers round each individual strand, were provided with a sputtered refractory aluminium oxide insulating layer, 5 μm thick, around the bundle of strands. The high temperature performance of twisted pairs of these wires was tested as described in Examples 1—6, and the results are shown in Table 5. From this table the benefits of the nickel intermediate layer are clear.

TABLE 5

| Example | Nickel intermediate layer thickness (μm) | Time to failure in a 900°C propane flame (min) |
|---|---|---|
| 1 (Comparison) | 0 | 0.2 |
| 17 | 3.7 | 21 |
| 18 | 9 | >150* |

* Testing stopped with no failure noted.

0 170 441

Examples 19 to 22

Wires similar to those in Examples 17 and 18, but provided with a sputtered aluminium additional layer, extending round the bundle of strands below the aluminium oxide layer, were also tested as described in Examples 1—6. The results are listed in Table 6. The benefits of the intermediate and additional metallic layers are illustrated.

TABLE 6

| Example | Nickel intermediate layer thickness (µm) | Aluminium additional layer thickness (µm) | Time to failure in a 900°C propane flame (min) |
|---|---|---|---|
| 19 | 0 | 1 | 2.5 |
| 20 | 1.3 | 0.5 | 23 |
| 21 | 2.7 | 0.5 | 44 |
| 22 | 9 | 0.5 | >300* |

* Testing stopped with no failure noted.

Examples 23 to 27

Copper wire samples similar to those in Examples 19—22, but with the aluminium additional layer made of sputtered nickel, were also tested as described in Examples 1—6. The results are presented in Table 7, which again highlights the benefits of the intermediate and additional layers on high temperature performance.

TABLE 7

| Example | Nickel intermediate layer thickness (µm) | Nickel additional layer thickness (µm) | Failure time in a 900°C propane flame (min) |
|---|---|---|---|
| 1 (Comparison) | 0 | 0 | 0.2 |
| 23 (Comparison) | 0 | 1.5 | 6 |
| 24 | 1.3 | 1.5 | 12 |
| 25 | 2.7 | 1.5 | 65 |
| 26 | 3.7 | 1.5 | 92 |
| 27 | 9 | 1.5 | >200* |

* Testing stopped with no failure noted.

## Claims

1. An electrical wire having a conductor which comprises a bundle of copper strands (1), the bundle having an adherent, electrically insulating, refractory coating (2) which extends around the circumference of the bundle but not around the individual strands (1), and the individual strands (1) having an intermediate layer (21) extending around them, the intermediate layer (21) being formed from a metal which acts as a barrier to diffusion of oxygen or copper or both and having a thickness of at least 1.5 micrometres.

2. A wire as claimed in Claim 1, wherein the intermediate layer (21) comprises aluminium, titanium, tantalum, chromium, manganese, nickel tin or silver.

3. A wire as claimed in claim 1 or claim 2, which includes an additional metallic layer (22) between the intermediate layer (21) and the refractory coating (22).

4. An electrical wire having a conductor which comprises a bundle of copper strands (1), the bundle having an adherent, electrically insulating, refractory coating (2) that extends around the bundle but not around the individual strands (1), and the individual strands (1) having an intermediate layer (21) extending around them, the intermediate layer being formed from a metal which acts as a barrier to diffusion of oxygen or copper or both, the conductor including an additional metallic layer (22) between the intermediate layer (21) and the refractory coating (2).

12

5. A wire as claimed in claim 3 or claim 4, wherein the additional layer (22) is formed from the same metal as that present in the refractory coating (2).

6. A wire as claimed in any one of claims 3 to 5, wherein the additional layer (22) comprises aluminium titanium, tantalum, chromium, manganese or nickel.

7. A wire as claimed in any one of claims 3 to 6, wherein the additional layer (22) extends around the circumference of the bundle but not around the individual strands (1).

8. A wire as claimed in claim 7, wherein the additional layer (22) comprises the same metal as is used for the intermediate layer (21).

9. A wire as claimed in claim 4, wherein the intermediate layer (21) has a thickness of at least 1 micrometre, preferably at least 1.5 micrometres and especially at least 2.0 micrometres.

10. A wire as claimed in any one of claims 1 to 9, wherein the refractory coating (2) comprises a metal oxide or nitride.

11. A wire as claimed in any one of claims 1 to 10, wherein the refractory coating (2) has been formed by a vacuum deposition process and preferably by a sputter plating method.

12. A wire as claimed in any one of claims 1 to 11, wherein the intermediate layer (21) is formed from a metal that forms an intermetallic compound or solid solution with copper when heated.

13. A wire as claimed in any one of claims 3 to 9, which contains more than one additional layer (22).

14. A wire as claimed in any one of claims 1 to 13, wherein the refractory coating (2) comprises aluminium oxide, silicon dioxide or aluminium nitride.

15. A wire as claimed in any one of claims 1 to 14, wherein the refractory coating (2) has a thickness of at least 1 micrometre preferably at least 2 micrometres and especially at least 5 micrometres.

16. A wire as claimed in any one of claims 1 to 15, wherein the refractory coating (2) has a stoichiometry that varies throughout at least part of its thickness such that the proportion of metal or semi-metal in the layer decreases toward the outer surface of the layer.

17. A wire as claimed in claim 16, wherein the stoichiometry of the refractory coating (2) varies such that there is no defined boundary between the intermediate layer and the refractory layer.

18. A wire as claimed in any one of claims 1 to 17, which has one or more additional layers (22) on top of the refractory coating (2).

19. A wire as claimed in any one of claims 1 to 18, which is provided with an additional outer polymeric insulation.

20. An electrical cable which comprises a bundle of wires as claimed in any one of claims 1 to 19.

**Patentansprüche**

1. Elektrischer Draht, der einen Leiter hat, der ein Bündel von Kupfersträngen (1) aufweist, wobei das Bündel einen klebenden, elektrisch isolierenden, schwer schmelzbaren Überzug (2) hat, der sich über den Umfang des Bündels, aber nicht um die einzelnen Stränge (1) erstreckt, wobei die einzelnen Stränge (1) eine Zwischenschicht (21) haben, die sich um dieselben erstreckt, und wobei die Zwischenschicht (21) aus einem Metall gebildet ist, das als eine Sperre für die Diffusion von Sauerstoff oder Kupfer oder beiden wirkt, und die eine Dicke von wenigstens 1,5 Mikrometer hat.

2. Draht nach Anspruch 1, bei dem die Zwischenschicht (21) Aluminium, Titan, Tantal, Chrom, Mangan, Nickel, Zinn oder Silber aufweist.

3. Draht nach Anspruch 1 oder Anspruch 2, der eine zusätzliche metallische Schicht (22) zwischen der Zwischenschicht (21) und dem schwer schmelzbaren Überzug (2) enthält.

4. Elektrischer Draht, der einen Leiter hat, der ein Bündel aus Kupfersträngen (1) aufweist, wobei das Bündel einen klebenden, elektrisch isolierenden, schwer schmelzbaren Überzug (2) hat, der sich um das Bündel, aber nicht um die einzelnen Stränge (1) erstreckt, wobei die einzelnen Stränge (1) eine Zwischenschicht (21) haben, die sich um dieselben erstreckt, wobei die Zwischenschicht aus einem Metall gebildet wird, das als eine Sperre für die Diffusion von Sauerstoff oder Kupfer oder beiden wirkt, und wobei der Leiter eine zusätzliche metallische Schicht (22) zwischen der Zwischenschicht (21) und dem schwer schmelzbaren Überzug (2) enthält.

5. Draht nach Anspruch 3 oder Anspruch 4, bei dem die zusätzliche Schicht (22) von dem selben Metall wie jenem gebildet wird, das in dem schwer schmelzbaren Überzug (2) vorhanden ist.

6. Draht nach einem der Ansprüche 3 bis 5, bei dem die zusätzliche Schicht (22) Aluminium, Titan, Tantal, Chrom, Mangan oder Nickel aufweist.

7. Draht nach einem der Ansprüche 3 bis 6, bei dem die zusätzliche Schicht (22) sich um den Umfang des Bündels, aber nicht um die einzelnen Stränge (1) erstreckt.

8. Draht nach Anspruch 7, bei dem die zusätzliche Schicht (22) das gleiche Metall wie jenes aufweist, das für die Zwischenschicht (21) verwendet wird.

9. Draht nach Anspruch 4, bei dem die Zwischenschicht (21) eine Dicke von wenigstens 1 Mikrometer, vorzugsweise wenigstens 1,5 Mikrometer, und insbesondere wenigstens 2,0 Mikrometer hat.

10. Draht nach einem der Ansprüche 1 bis 9, bei dem der schwer schmelzbare Überzug (2) ein Metalloxid oder -nitrid aufweist.

11. Draht nach einem der Ansprüche 1 bis 10, bei dem der schwer schmelzbare Überzug (2) durch ein Vakuumbedampfungsverfahren und vorzugsweise durch ein Zerstäubungsplattierungsverfahren gebildet wurde.

12. Draht nach einem der Ansprüche 1 bis 11, bei dem die Zwischenschicht (21) aus einem Metall gebildet wird, das bei der Erwärmung eine intermetallische Verbindung oder eine feste Lösung mit Kupfer bildet.

13. Draht nach einem der Ansprüche 3 bis 9, der mehr als eine zusätzliche Schicht (22) enthält.

14. Draht nach einem der Ansprüche 1 bis 13, bei der der schwer schmelzbare Überzug (2) Aluminiumoxid, Siliziumdioxid oder Aluminiumnitrid aufweist.

15. Draht nach einem der Ansprüche 1 bis 14, bei dem der schwer schmelzbare Überzug (2) eine Dicke von wenigstens 1 Mikrometer, vorzugsweise wenigstens 2 Mikrometer und insbesondere wenigstens 5 Mikrometer hat.

16. Draht nach einem der Ansprüche 1 bis 15, bei dem der schwer schmelzbare Überzug (2) eine Stöchiometrie hat, die sich wenigstens über einen Teil der Dicke hinweg derart ändert, daß der Anteil des Metalls oder Halbmetalls in der Schicht in Richtung zur äußeren Fläche der Schicht abnimmt.

17. Draht nach Anspruch 16, bei dem die Stöchiometrie des schwer schmelzbaren Überzugs (2) sich derart ändert, daß keine definierte Grenze zwischen der Zwischenschicht und der schwer schmelzbaren Schicht vorhanden ist.

18. Draht nach einem der Ansprüche 1 bis 17, der eine oder mehr zusätzliche Schichten (22) auf dem schwer schmelzbaren Überzug (2) hat.

19. Draht nach einem der Ansprüche 1 bis 18, der mit einer zusätzlichen äußeren polymeren Isolierung versehen ist.

20. Elektrisches Kabel, das ein Bündel aus Drähten gemäß einem der Ansprüche 1 bis 19 aufweist.

**Revendications**

1. Fil électrique ayant un conducteur qui comporte un groupe de brins (1) de cuivre, le groupe ayant un revêtement réfractaire adhérent et isolant de l'électricité (2) disposé autour de la circonférence du groupe mais non autour des brins individuels (1), et les brins individuels (1) ayant une couche intermédiaire (21) disposée autour d'eux, la couche intermédiaire (21) étant formée d'un métal qui constitue une barrière s'opposant à la diffusion d'oxygène, de cuivre ou des deux et ayant une épaisseur au moins égale à 1,5 μm.

2. Fil selon la revendication 1, dans lequel la couche intermédiaire (21) est formée d'aluminium, de titane, de tantale, de chrome, de manganèse, de nickel, d'étain ou d'argent.

3. Fil selon l'une des revendications 1 et 2, qui comporte une couche métallique supplémentaire (22) placée entre la couche intermédiaire (21) et le revêtement réfractaire (2).

4. Fil électrique ayant un conducteur qui comporte un groupe de brins (1) de cuivre, le groupe ayant un revêtement réfractaire adhérent et isolant de l'électricité (2) disposé autour du groupe mais non autour des brins individuels (1), et les brins individuels (1) ayant une couche intermédiaire (21) disposée autour d'eux, la couche intermédiaire étant formée d'un métal qui joue le rôle d'une barrière s'opposant à la diffusion de l'oxygène, du cuivre ou des deux, le conducteur comprenant une couche métallique supplémentaire (22) placée entre la couche intermédiaire (21) et le revêtement réfractaire (2).

5. Fil selon l'une des revendications 3 ou 4, dans lequel la couche supplémentaire (22) est formée du métal qui est présent dans le revêtement réfractaire (2).

6. Fil selon l'une quelconque des revendications 3 à 5, dans lequel la couche supplémentaire (22) est formée d'aluminium, de titane, de tantale, de chrome, de manganèse ou de nickel.

7. Fil selon l'une quelconque des revendications 3 à 6, dans lequel la couche supplémentaire (22) est disposée autour de la circonférence du groupe mais non autour des brins individuels (1).

8. Fil selon la revendication 7, dans lequel la couche supplémentaire (22) contient le métal qui est utilisé pour la couche intermédiaire (21).

9. Fil selon la revendication 4, dans lequel la couche intermédiaire (21) a une épaisseur au moins égale à 1 μm et de préférence au moins égale à 1,5 μm et tout particulièrement au moins égale à 2,0 μm.

10. Fil selon l'une quelconque des revendications 1 à 9, dans lequel le revêtement réfractaire (2) est un oxyde ou nitrure métallique.

11. Fil selon l'une quelconque des revendications 1 à 10, dans lequel le revêtement réfractaire (2) a été formé par un procédé de dépôt sous vide et de préférence par un procédé de revêtement par pulvérisation cathodique.

12. Fil selon l'une quelconque des revendications 1 à 11, dans lequel la couche intermédiaire (21) est formée d'un métal qui forme un composé intermétallique ou une solution solide avec le cuivre, lorsqu'il est chauffé.

13. Fil selon l'une quelconque des revendications 3 à 9, qui contient plus d'une couche supplémentaire (22).

14. Fil selon l'une quelconque des revendications 1 à 13, dans lequel le revêtement réfractaire (2) est l'oxyde d'aluminium, le bioxyde de silicium ou le nitrure d'aluminium.

15. Fil selon l'une quelconque des revendications 1 à 14, dans lequel le revêtement réfractaire (2) a une épaisseur au moins égale à 1 μm, de préférence au moins égale à 2 μm et tout particulièrement au moins égale à 5 μm.

14

16. Fil selon l'une quelconque des revendications 1 à 15, dans lequel le revêtement réfractaire (2) a une composition stoechiométrique qui varie dans une partie au moins de son épaisseur de manière que la proportion du métal ou semi-métal de la couche diminue vers la surface externe de la couche.

17. Fil selon la revendication 16, dans lequel la composition stoechiométrique du revêtement réfractaire (2) varie de manière qu'il n'existe pas de limite bien définie entre la couche intermédaire et la couche réfractaire.

18. Fil selon l'une quelconque des revendications 1 à 17, qui a une ou plusieurs couches supplémentaires (22) placées sur le revêtement réfractaire (2).

19. Fil selon l'une quelconque des revendications 1 à 18, qui comporte un isolement polymère externe supplémentaire.

20. Câble électrique qui comporte un groupe de fils selon l'une quelconque des revendications 1 à 19.

Fig.1.

Fig.3

FIG.2

# Fig.4.